# EUROPEAN PATENT SPECIFICATION

(11) **EP 2 229 918 B1**
(45) Date of publication and mention of the grant of the patent: **24.07.2013**
(21) Application number: 10157056.2
(22) Date of filing: 19.03.2010
(51) Int. Cl.: A61F 2/00

(54) **Surgical implant, in particular for management of hernias**
Chirurgisches Implantat, insbesondere zur Hernienbehandlung
Implant chirurgical, en particulier pour la gestion des hernies

(30) Priority: 19.03.2009 DE 102009015302
(43) Date of publication of application: 22.09.2010
(73) Proprietor: Aesculap AG, 78532 Tuttlingen/Donau (DE)
(72) Inventor: Odermatt, Erich, 8200, Schaffhausen (CH); Abele, Wolfgang, 78532, Tuttlingen (DE); König, Silke, 78628, Rottweil (DE); Müller, Erhard, 70565, Stuttgart (DE); Schmees, Hans-Gerd, 72827, Wannweil (DE)
(74) Representative: Patentanwälte Ruff, Wilhelm, Beier, Dauster & Partner

(56) References cited:
- WO-A2-2007/120138
- US-A1- 2001 056 303
- US-A1- 2002 052 649
- US-A1- 2002 077 661
- US-A1- 2002 116 070
- US-A1- 2003 236 575

## Description

The present invention relates to a surgical implant which is especially useful for hernia management, and also to a method of forming the implant.

A hernia in the groin region is a protrusion (hernial sac) of the peribneum through a congenital or acquired gap known as the hernial aperture. When standing and particularly when straining, coughing or sneezing, loops of bowel generally will pass into the hernial sac and form a palpable and/or visible lump which generally is easy to push back into the abdominal cavity when lying down. The commonest forms of external hernias, where the hernial sac is always enveloped by the peribneum, are inguinal, umbilical and incisional hernias. The main reasons for hernias are muscular or connective tissue infirmities associated with overexertion, age-related atonia, insufficient scar formation following a surgical intervention, or a congenital weakness in the abdominal wall.

Hernia management typically involves a surgical procedure in which the contents of the hernial sac are put back in the abdomen and the hernial orifice is subsequently closed. Closing the hernial orifice was hitherto conventionally done using suture materials. However, this method of closing leads again and again, particularly in the case of relatively large hernias, to the hernia reoccurring.

This is why polymeric nets have come to be increasingly used to manage relatively large hernias in particular. These polymeric nets can be used both for open surgery and for minimally invasive surgery (MIS). In open surgery, generally, an incision 5 to 10 cm in length is made in the groin region and all the layers of the abdominal wall are prepared before the net is implanted. The so-called Lichtenstein operation is very commonly used. In this surgical method, the net is implanted between the inner and outer abdominal muscle or facia.Another technique commonly used is the so-called Stoppa operation, where the net is implanted between the peritoneum and the inner abdominal wall, making it easy to remove it again in the event of a rejection reaction.

The recent trend is more and more to the use of the previously mentioned minimally invasive surgical techniques. These offer appreciably more patient comfort and confidence in load-bearing capacity compared with the conventional open surgical methods. The so-called TEP technique is one example of minimally invasive surgery. It generally involves an abdominal wall laparoscopy being performed minimally invasively or endoscopically via three small incisions (5 to 10 mm). This intervention is extremely tissue-conserving in that the abdominal musculature, which contains nerves, and also the peritoneum are neither cut nor stitched. In the so-called TAPP technique, the minimally invasive mesh placement is via the abdominal cavity. For this, the peritoneum has to be cut open and subsequently closed again by stitching. Both in TEP and in TAPP, the mesh is to some extent fixed by the abdominal internal pressure and the counterpressure of the abdominal musculature.

However, irrespectively of the particular method of surgery used, it is necessary to additionally fix the implanted mesh because otherwise there is too much of a risk that the net may become displaced and the hernia reoccurs. In general, the nets are fixed by means of suitable staples or suture materials. Fixing usually tends to be very difficult and inconvenient in both open and closed surgical techniques. One reason for this is that the edges of the mesh generally lie between tissue layers, which make mesh fixing additionally difficult. In addition, the use of staples or sutures, as artificial fixing means, represents an additional incorporation of material, and can lead to inflammatory reactions and also additional tissue trauma.

Biocompatible implants constructed of a knitted pile mesh material comprising a plurality of filament extensions are known from US 2002/0116070 A1. Percutaneous skin access devices including a plurality of tissue locking configurations in the form of loops, rings or hooks are known from US 2003/0236575 A1.

It is an object of the present invention to provide a selfretaining implant which avoids the inadequacies described in the prior art and more particularly reduces the risk of inflammatory reactions and additional tissue trauma compared with conventional implants.

This object is achieved by a surgical implant having the features of independent claim 1. Preferred embodiments of the implant of the present invention form part of the subject matter of dependent claims 2 to 14. The present invention also provides a method of forming the implant of the present invention as per independent claim 15.

The implant according to the present invention comprises a surgical implant in the form of a textile fabric having at least two, in particular two, main surfaces, preferably mutually opposite main surfaces, more particularly for management of hernias, comprising threads having an elongate body and having anchoring structures, in particular a multiplicity of anchoring structures, which preferably project from the body of the threads, for anchoring in biological, more particularly human and/or animal, tissues.

In principle, all threads of the textile fabric may have anchoring struttures, which preferably project from the thread bodies. However, it is more preferable that the threads of the textile fabric only in part may comprise anchoring structures. In other words, it is preferred that the textile fabric additionally comprises threads that are free of anchoring structures that are envisaged by the present invention.

A multiplicity of anchoring structures herein is preferably to be understood as meaning at least 2 anchoring structures projecting from the thread surface. For example according to the present invention it may be envisaged that at least 2, more particularly at least 10, preferably between 2 and 100, and at most preferably between 10 and 100, anchoring structures are present per cm² thread surface.

The anchoring structures provided according to the present invention, which are formed on the thread bodies, bore their way through fine, low-irritation puncture channels into a biological tissue and thereby provide secure anchoring/retention of the implant in the region of a surgical application. The anchoring structures advantageously facilitate a physical, in particular mechanical and/or adhesive, adherence of the implant, in particular of at least one main surface thereof, to biological tissue surfaces, in particular soft tissue surfaces. In other words, the present invention provides with particular advantage a self-retaining implant. The use of additional fixing means, more particularly staples, suture materials or tissue adhesives, is not required and hence no additional introduction of material is needed either. Because no additional material is introduced into the body of the patient, it is possible to significantly reduce the risk of inflammatory reactions and also tissue trauma. A further advantage is that the anchoring security of the implant can be influenced specifically via the number of anchoring structures. Because no additional fixing means are needed for the implant, it is also more convenient and simpler to implant by means of endoscopic, more particularly laparoscopic, methods.

In a preferred embodiment, the threads, which have the anchoring structures, or parts of these threads, preferably at least in part, project from the textile fabric. The threads and parts thereof respectively can in turn like the anchoring structures, bore their way into a tissue and thereby additionally improve the anchoring possibilities for the implant. The threads, which have the anchoring structures, or parts of these threads together with the anchoring structures advantageously enhance the strength and security of the anchoring of the implant. Therefore, the risk of a possible displacement of the implant from its oiginal implanted position may be reduced. Preferably, the projecting threads having the anchoring structures or parts of these threads are firmly fixed with the textile fabric.

In a further preferred embodiment, the threads having the anchoring structures or parts of these threads, preferably at least partially, project from the textile fabric in the form of loops, in particular velour loops, floats and/or overdimensioned threads. In the case of loops, medium to long-term anchoring can be effected throughingrowth of cells, in particular body cells.

In a further preferred embodiment, the threads, which have the anchoring structures, or parts of these threads, preferably at least in part, poject from the textile fabric as cut piles, cut pile tufts, pile yarns, plush or combinations thereof. More preferably, the threads or parts thereof, preferably at least partially, project from the textile fabric in the form of opened velour loops.

In a further embodiment, the projecting threads or parts of these threads are tightly or firmly connected with the textile fabric, preferably with at least one main surface thereof, in particular both main surfaces thereof. This has the advantage that the projecting threads and parts of these threads respectively cannot be displaced or removed from the textile fabric or main surfaces thereof. Als already mentioned, the projecting threads and parts thereof respectively may be designed as loops, in paticular velour loops, floats, overdimensioned threads, cut piles, cut pile tufts, in particular opened velour loops, pile yarns, plush or combirations thereof.

The threads, which have the anchoring structures, or parts of these threads, preferably at least partially, project from at least one of the two main surfaces of the textile fabric. In principle, the threads or parts thereof may project from both main surfaces of the textile fabric. With respect to special applications of the implant, it is also preferably that the threads or parts thereof only project from one main surface of the textile fabric. For instance, when the implant is used for the management of hernias, the main surface with the projecting threads and parts thereof respectively is preferably provided as contact surface with the abdominal wall. The opposite main surface is preferably free from projecting threads or parts thereof in order to avoid post-surgical tissue adhesions in respect of the abdominal cavity.

It is further within the scope of the present invention that the implant may comprise loops, in particular velour loops, floats, overdimensioned yarns, cut piles, cut pile tufts, preferably opened velour loops, pile yarns or a combination thereof, which are free from anchoring structures.

The threads, which have the anchoring structures, or parts of these threads may project from the textile fabric, in particular from at least one of the main surfaces, in a row-shaped arrangement, in an offset a-rangement, island-like arrangement, in a zigzag-shaped arrangement, in a spiral-shaped arrangement, in a serpentine-shaped arrangement, in a meander-shaped arrangement, an adventitious arrangement and/or a random arrangement. Furthermore, the threads or parts thereof may project at the edges, more particularly only at the edges, of the textile fabric.

The threads, which have the anchoring structures, or parts of these threads can have a length between 0.5 and 2.5 mm, more particularly 0.5 and 0.8 mm.

In a further embodiment, the threads having the anchoring structures, or parts of these threads project in a density between 10 and 400 per cm², more particularly 30 and 400 per cm², preferably 40 and 200 per cm², from the textile fabric, more particularly from at least one of the two main surfaces thereof.

In principle, the threads having the anchoring structures or parts of these threads can project from the textile fabric at any desired angle. However, in accordance with the teaching of the present invention, it is preferable when the threads or parts of these threads project at essentially right angles from the textile fabric.

In a further embodiment, the anchoring structures of projecting threads or parts thereof are aligned unidirectionally in the direction of the textile fabric, more particularly in the direction of a main surface thereof. This makes it possible to further reduce tissue trauma when the projecting threads or thread parts bore their way into the tissue, for example into the tissue of the abdominal wall.

In a further embodiment, the anchoring structures are formed in a hook, more particularly barb, escutcheon, shield, scale, wedge, spike, dart, harpoon, arrow, V and/or W shape on the elongate body of the threads. The anchoring structures form barbs on the thread bodies. Furthermore, the anchoring structures may be pointed/sharp at their distal end.

In a further embodiment, the anchoring structures may have a substantially rectilinear body and, on the free end of said body, a head with a length preferably greater than that of the rectilinear body.

The anchoring structures are formed as cuts on the thread bodies. The cuts may be mechanical, thermal, physical-chemical or laser produced. The formation of such cuts will be more particularly discussed hereinbelow.

According to the present invention, the anchoring structures can have a cut depth, measured perpendicularly from the thread surface, between 5 and 50%, more particularly 15 and 45% and preferably 20 and 35% of the diameter of the threads without anchoring structures (diameter of threads in uncut state). In a further embodiment, the anchoring structures project from the thread bodies at an angle between 5 and 80 degrees, more particularly 10 and 70 degrees and preferably 15 and 60 degrees.

Furthermore, the anchoring structures may be configured in different arrangements on the thread bodies. For example, the anchoring structures may have a row-shaped arrangement, an offset arrangement, island-like arrangement, a zigzag-shaped arrangement, a spiral-shaped arrangement, a serpentine-shaped arrangement, a meander-shaped arrangement, an adventitious arrangement, a random arrangement or combinations thereof, in the longitudinal and/or transverse direction, preferably in the longitudinal direction, on the thread bodies. Particular preference is given to an arrangement in which the anchoring structures are distributed over the entire surface of the thread body, since in this way a particularly firm and secure anchoring of the implant in a biological tissue can be achieved.

An island-like arrangement of the anchoring structures, in particular on at least one main surface of the textile fabric, may be especially preferred in order to facilitate a fixation of the implant to a biological tissue surface.

In a further embodiment, the anchoring structures are formed uni- or bidirectionally on the thread bodies. A unidirectional arrangement of the anchoring structures herein is to be understood as meaning an arrangement in which the anchoring structures are uniformly oriented in one direction. By contrast, a bidirectional arrangement of anchoring structures is to be understood as referring to an arrangement in which the anchoring structures on the thread bodies are oriented in two different directions. In the case of a bidirectional arrangement, it is preferable when the anchoring structures, when viewed in the longitudinal direction of the threads, are formed in a first portion of the thread to face in the direction of a remaining second portion of the thread and in the remaining second portion of the thread toface in the direction of the first portion of the thread. According to the present invention, a change in the orientation of the anchoring structures can take place at the thread midpoint for example.

In a further embodiment, the anchoring structures on the thread bodies have a periodically repeating, more particularly alternating, orientation. Thus, viewed in the longitudinal direction of the threads, the anchoring structures may be formed in a first portion of the thread to face in the direction of a second portion of the thread and in the second portion of the thread to face in the direction of the first portion of the thread, in a third portion of the thread, which adjoins the second portion of the thread, to face in the direction of a fourth portion of the thread, which adjoins the third portion of the thread, and in the fourth portion of the thread to face in the direction of the third portion of the thread, and so on.

In a further embodiment, the threads each include at least one set, more particularly two, three or more sets, of anchoring structures. A set of anchoring structures herein is to be understood as referring to an arrangement of anchoring structures on the thread bodies which in relation to the configuration of the anchoring structures, more particularly in relation to the height of the anchoring structures, the length of the anchoring structures, the cut depth of the anchoring structures, angles between the anchoring structures and the thread bodies, alignment of the anchoring structures and/or form/shape of the anchoring structures, is coincident. Preferably, two, three, four, five, six or more sets of anchoring structures are formed in the circumferential direction of the threads. The larger the number of such sets formed in the circumferential direction of the threads, the greater the strength and security possible for an anchoring of the implant via the anchoring structures.

The threads can in principle be formed of any polymeric material suitable for forming surgical threads. The polymeric materials may comprise absorbable, semiabsorbable or nonabsorbable materials. The polymeric materials may further be present as homo-, co-, ter- or tetrapolymers and so on. Examples of suitable polymeric materials are block polymers, more particularly block copolymers or block terpolymers. The use of random/adventitious or alternating co-, ter- or tetrapolymers and so on is likewise possible according to the present invention.

In a further embodiment, the textile fabric, in particular threads thereof, preferably the threads having the anchoring structure, is formed of an absorbable polymeric material. The polymeric material may be a synthetic or natural occurring polymer. More particularly, the polymeric material is selected from the group consisting of polylactide, polyglycolide, poly-ε-caprolactone, poly-para-dioxanone, polytrimethylene carbonate, polyhydroxybutyrate, in particular poly-3-hydroxybutyrate and/or, poly-4-hydroxybutyrate, polyvinyl alcohol, copolymers thereof, terpolymers thereof and combinations thereof. Preference is given to the use of copolymers or terpolymers, more particularly block copolymers or block terpolymers, including at least one monomer from the group consisting of lactide, glycolide, trimethylene carbonate, paradioxanone, ε-caprolactone and hydroxybutyrate. Hydroxybutyrate may be 3-hydroxybutyrate and/or 4-hydroxybutyrate. Suitable natural occurring polymer materials may be selected from the group consisting of proteins and polysaccharides, in particular mucopolysaccharides, salts thereof and combinations thereof.

In a further embodiment, the textile fabric, in particular threads thereof, preferably the threads having the anchoring structures, is formed of an nonabsorbable polymeric material, preferably selected from the group consisting of polyolefins, polyesters, polyamides, polyurethanes, copolymers thereof, terpolymers thereof and combinations thereof. Sutable nonabsorbable polymeric materials are more particularly polyethyene, polypropylene, polytetrafluoroethylene, in particular expanded polytetrafluoroethylene, polyvinylidene difluoride, polytetrafluoropropyene, polyhexafluoropropylene, polyethyleneterephthalate, polybutyleneterephthalate, copolymers thereof, terpolymers thereof or combirations thereof.

It is further within the scope of the present invention that the textile fabric, in particular threads thereof, preferably the threads having the anchoring structure, is made of a semiabsorbable material, particularly a semiabsorbable polymer material. More specifically, the textile fabric, in particular at least one main surface thereof, may be made of absorbable and nonabsorbable materials, in particular polymer materials. In this regard, reference is made entirely to the previous description.

Furthermore, it is within the present invention that at least one of the main surfaces, in particular both main surfaces, of the textile fabric is made of a material, in particular polymer material, as described in the previous embodiments.

In principle, the anchoring structures may be made from the same material like the threads from which the anchoring structures preferably project. However, it is also within the scope of the present invention that the anchoring structures may be made from a different material than the threads from which they preferably project. For example, the anchoring structures can be made of a swellable material, preferably based on a polymer which soaks water and body fluids, in particular wound fluids. Examples of suitable polymers may be selected from the group consisting of starch, amylose, amylopectine, dextrane, chitosan, cellulose, methylcellulose, carboxymethylcellulose and mucopolysaccarides, in paticular hyaluronic acid, salts thereof and combinations thereof.

In a preferred embodiment, the threads having the anchoring structures are present together with other threads which have no anchoring structures in a conjoined textile construction. The textile construction may be selected here from the group consisting of the Atlas, filet, tulle, mesh and openwork constructions. It can further be provided according to the present invention that the threads having the anchoring structures provided according to the present invention and threads not having such anchoring structures alternate in a repeat pattern.

In a further embodiment, the textile fabric is formed as a woven fabric, a non-woven fabric, a felt, a knit fabric, in particular loopformingly knit fabric or a braided fabric. However, it is preferable according to the present invention when the textile fabric is formed as a formed-loop knit fabric, more particularly warp knit fabric, in particular formed-looped warp-knit fabric, or braid. The textile fabric further preferably has a porous structure, in particular a net-like or mesh-like structure. Preferably the textile fabric is designed as a textile net or mesh.

It is further preferable that at least one main surface, in particular both main surfaces or as an alternative only one main surface of the textile fabric is designed as a non-woven fabric, a felt, a knit fabric, in particular loop-formingly knit fabric, particularly a warp knit fabric, more preferably formed-loop warp-knit fabric, or a braided fabric. The design as a textile net or mesh is especially preferred.

In a further embodiment, the textile fabric, in particular a main surface thereof, comprises a coating, film or foil, preferably with anti-adhesive properties. More specifically, the coating, film or foil may have a flat, smooth and/or tight structure. The coating or film may be designedas a uniform and in particular sealing coating or film. More preferably the coating or film is formed as holohedral coating or film. Preferably, the foil possesses a mesh-like structure. In order to achieve an anti-adhesive effect towards tissue surfaces, it is within the scope of the present invention that the coating, film or foil may be made of a hydrophobic material like polytetrafluoroethylene, in particular expanded polytetrafluooethylen, or polypropylene, and/or, where applicable, may be treated with fluorine or chlorine.

In a preferred embodiment, the coating or film as mentioned above is preferably formed of an absorbable material. It is particularly advantageous for the coating or film material to have an absorption time of at least 7 days. Thereafter, new peritoneum, known as pseudoperitoneum, will generally have formed over the implant, and can then take over the function of the coating and act as a natural protective barrier against possible tissue adhesions in respect of the abdominal cavity. Suitable materials for the coating or film are synthetic and/or natural polymers. Examples of synthetic polymers are polyesters, more particularly polyhydroxyalkanoates, more particularly selected from the group consisting of polyglycolide, polylactide, pdy-ε-caprolactone,polytrimethylene carbonate, poly-para-dioxanone, polyhydroxybutyric acid, polyvinylalcohol (PVA) copolymers thereof, terpolymers thereof and combinations thereof. Preferred natural polymers are selected from the group consisting of albumin, elastin, reticulin, fibronectin, collagen, gelatin, starch, dextrane, cellulose, methylcellulose, carboxymethylcellulose, mucopolysaccarides, in particular hyaluronic acid, derivatives thereof, salts thereof and mixtures thereof. The coating or film material may be in a crosslinked state, more particularly in a chemically crosslinked state, or in an uncrosslinked state. When gelatin is used as coating or film material, it can have a molecular weight of 50 to 200 kDa (kilodaltons), more particularly about 100 kDa. Gelatin may be chemically denatured collagen or thermally denatured collagen.

In a particularly preferred embodiment, the threads, which have the anchoring structures, or parts of these threads, preferably at least partially, only project from one of the two main surfaces while the other main surface comprises a coating, film or as an alternative a foil as described in the previous embodiments. The main surface with the projecting threads or parts thereof preferably faces the abdominal wall afterimplantation of the implant, in which case a secure anchoring of the implant can be offected through the thread and parts thereof respectively and also the anchoring structures which preferably project therefrom boring their way into the tissue of the abdominal wall. After implantation, the opposite main surface comprising the coating, film or foil preferably faces the abdominal cavity. The coating, film or foil may act with particular advantage as a kind of barrier which prevents any post-surgical intergrowth between soft tissue of the abdominal cavity and the implant.

In a further embodiment, the threads, which have the anchoring structures, or parts of these threads interconnect the two main surfaces of the textile fabric.

In a preferred embodiment, the textile fabric may be designed as a spacer textile fabric, in particular spacer knit fabric, preferably a spacer warp knit fabric. The spacer fabric in turn may be composed of at least two, in particular two, textile entities which may be interconnected by textile means like fibres, threads, yarns or combinations thereof. Each textile entity has two, preferably mutually opposite, main surfaces. In other words, in this embodiment the textile fabric (being designed as spacer textile fabric) has at least four, preferably mutually opposite, main surfaces. Preferably, the textile entities are interconnected by means of the threads according to the present invention, which have anchoring structures preferably projecting from the thread bodies. More preferably, the textile entities are interconnected by the anchoring structures according to the present invention. In other words, the anchoring structures that are envisaged by the present invention may be used to fix textile entities to each other, preferably in order to form a spacer textile fabric. This is advantageously with respect to the management of inguinal hernias allowing for a tension free placement of the spacer textile fabric around the spermatic cord. In principle, the textile entities may have the same or a different textile construction. The textile entities may be selected from the group consisting of a woven entity, a non-woven entity, a felt, a knitted entity, in particular loop-formingly knitted entity, a warp knitted entity, a braided entity and a textile mesh. By way of example, both textile entities may be designed as a mesh. As an alternative, one textile entity may be designed as a mesh, whereas the other textile entity is designed as a foil or felt. It is also within the scope of the present invention that the textile entities may be composed of different meshes. Furthermore, the textile entities and the afore-mentioned interconnecting textile means may be made of the same material or of a different material, in particular polymer material. Regarding further details of the material and polymer material respectively reference is made entirely to the previous description.

In a further embodiment, the textile fabric, in particular at least one main surface thereof, has a pore size between 0.3 and 6µm, more particularly 0.3 and 5 µm, preferably 0.8 and 4µm. It can further be provided according to the present invention for the textile fabric to have a basis weight between 25 and 85 g/m². Preference is given to basis weights between 30 and 70 g/m², more particularly 35 and 50 g/m². Lightweight implants have the basic advantage that unwanted rejections and scarring are more likely avoidable than in the case of heavyweight implants.

In a further embodiment, the fabric is formed of monofil and/or multifil, preferably monofil, threads. The monofil threads may in principle also be pseudomonofil threads. In general, the threads have a circular cross section. However, other cross-sectional shapes are also conceivable in principle. For example, the threads can have an oval, triangular/trilobal, square, trapezoidal, rhomboid, pentagonal/five-cornered, hexagonal/six-cornered, star-shaped or cruciform cross section. Such cross-sectional shapes are readily realized with the aid of corresponding extrusion dies which can be custom made to any desired cross-sectional shape. The threads may further have a thickness between 80 and 250 µm, preferably 100 and 200 µm.

In a further embodiment, the implant, in particular threads of the implant, include additives like antimicrobial, in particular antibiotic, substances, disinfecting substances, anti-inflammatory substances, wound healing promoting substances, pain-killing substances, cellular growth factors, cellular differentiating factors, cellular adhesion factors, cellular ecruiting factors, anesthetic substances, swellable additives or combinations thereof. Swellable additives like superadsorbers, hydrogels and so on are preferable in order to strengthen the adhesive force of the implant towards tissue surfaces, in particular soft tissue surfaces.

In a further embodiment, the implant, in particular threads thereof, may be coated with additives, in particular additives as mentioned in the pevious embodiment.

In a further embodiment, the implant is present in sterilized and more particularly packaged form. The customary physical or chemical methods of inactivating microorganisms are sulable for sterilization. One possible method of sterilization involves treatment with ionizing radiation, particularly with β-rays or γ-rays. Doses of radiation suitable for this can be in the range between 5 and 38kGy. Gas sterilization, for example using ethylene oxide or formaldehyde, is a further suitable method of sterilization. Other possible methods of sterilzation involve a plasma sterilization or steam treatment of the implant.

The implant of the present invention is particularly useful in surgery, more particularly for treating soft tissue defects and/or wall defects in body cavities, for example for treating abdominal-wall, thorax and/or abdominal defects like hernias, in particular incisional hernias, inguinal hernias, and/or prolapses. It is therefore preferable according to the pesent invention for the implant to be configured as a hernia net, urine incontinence net or prolapse net. Configuration as a hernia net, more particularly a self-retaining hernia net, is particularly preferred according to the present invention. A further field of application for the implant according to the present invention relates to wound closure, in particular to adaptation of mutually opposite sides of a wound.

A further aspect of the present invention relates to a method of forming the implant. To this end, a textile fabric is formed using, preferably at least in part, threads having an elongate body and having anchoring structures which preferably project from the elongate body.

In a preferred embodiment, as the textile fabric forms, at least some of the threads are led out of it.

In an alternative embodiment, in a first step the textile fabric is designed as a spacer textile fabric having preferably two mutually opposite textile entities which are interconnected by the threads having anchoring structures and in a second step the spacer textile fabric is cut between the two textile entities yielding textile structures having projecting threads or parts thereof projecting from one main surface of the textile structures. The cutting may be performed by means of heat treatment, laser treatment or mechanical cutting. The projecting threads or parts thereof may be formed as cut piles, cut pile tufts, plush, pile yarns or combinations thereof.

In an alternative embodiment, the threads which have the anchoring structures are incorporated in a prefabricated textile fabric and at least some of the threads are led back out of the prefabricated textile fabric.

Preferably, the threads led out form loops, more particularly velour loops, floats and/or overdimensioned threads, as they are led back into the textile fabric. The loops are generally opened, preferably severed, to form projecting threads or thread parts.

The anchoring structures envisaged according to the present invention can be formed by being cut into the threads. The cutting of the anchoring structures can be effected in the drawn or undrawn state of the threads. Furthermore, the threads can be in a twisted or untwisted state during the cutting of anchoring structures. When the threads are in a twisted state during the cutting of anchoring structures, this is a particularly simple way of producing spiral-shaped arrangements of anchoring structures on the thread bodies. According to the present invention, the anchoring structures can be cut into the threads to a cut depth, measured perpendicularly from the thread surface, which is between 5 and 50%, more particularly 15 and 45% and preferably 20 and 35% of the diameter of the threads without anchoring structures.

To form the anchoring structures envisaged according to the present invention, these can be cut mechanically into the thread bodies for example. Customary cutting devices can be used for this purpose, most simply a cutting blade. Suitable cutting devices generally comprise a cutting bed, at least one cutting blade and also holding or retaining elements, for example a vice, chucks, holding or clamping jaws, for the threads into which cuts are to be made. A further way of forming the anchoring structures envisaged according to the present invention consists in cutting into the thread bodies thermally. Thermal cutting into the thread bodies has the advantage that the cut ends in the thread bodies which are produced by thermal cutting are less tapered, more particularly less acute, than result from cutting mechanically into the thread bodies. This can be used to minimize the risk of the threads developing a tear starting from the respective cut ends, for example in the course of subsequent drawing of the threads or under other loads, for example under pressures of the kind that can typically occur in the body of a patient. Thermal cutting can be effected using a heated cutting wire for example.

Alternatively to the above-described cutting techniques, the anchoring structures can also be formed on the thread bodies by laser cutting techniques. Appropriate techniques are sufficiently familiar to a person skilled in the art, which is why more detailed observations are dispensed with here.

For further details and advantages of the inventive method reference is made entirely to the total description.

Finally, the present invention also provides for the use of threads having an elongate body and having a multiplicity of anchoring structures generally projecting from the elongate body for manufacturing a surgical implant, preferably a self-retaining surgical implant. The implant is configured as a textile net and more particularly as a hernia net, urine incontinence net or prolapse net. With regard to further features and details thereto, reference is made to the description heretofore in its entirety.

Further features of the invention will become apparent from the following description of preferred embodiments in conjunction with the figures and the subclaims. Individual features can be actualized either singly or plurally in combination with each other.

In the figures are schematically shown:

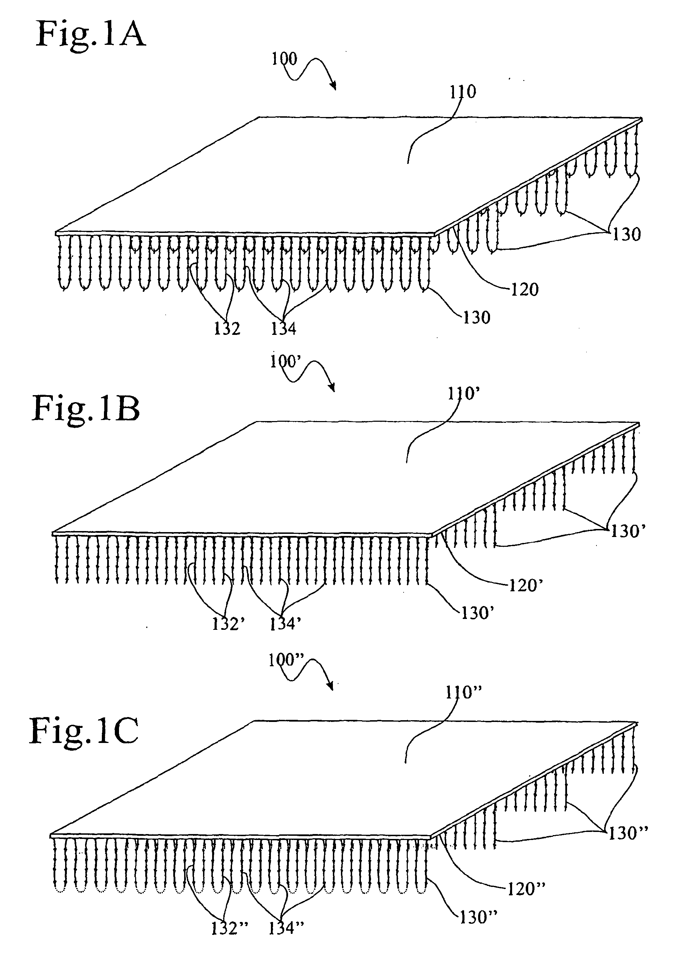

- Fig. 1A:: an embodiment of the inventive implant,
- Fig. 1B:: a further embodiment of the inventive implant,
- Fig. 1C:: a further embodiment of the inventive implant.

### Figure description

The surgical implant 100 depicted in Fig. 1A is formed as a textile fabric. The textile fabric may be a woven fabric, an interlooped fabric, a knit fabric, in particular a formed-loop knit fabric, more preferably a warp knit fabric, or a braid. The implant has two main surfaces 110 and 120, which are opposite each other. Main surface 110 preferably has a smooth flat and/or tight construction. For example, main surface 110 can have a smooth and more particularly sealing coating. Useful coating materials include for example natural polymers, more particularly selected from the group consisting of albumin, elastin, reticulin, fibronectin, collagen, gelatin, derivatives thereof and mixtures thereof. The preferably smooth construction of the main surface 110 makes it possible to avoid, with particular advantage, undesirable tissue adhesions on this side of the implant 100. For example, a smoothly constructed main surface 110 following an intraperitoneal implantation may face the abdominal cavity of a patient. When the smooth construction of the main surface 110 is attributable to a coating, it is particularly advantageous for the coating material to have an absorption time of at least 7 days. Within this period, a new peritoneum, a so-called pseudoperitoneum, will generally form over the main surface 110 and then be able to perform the barrier function of a coating.

The main surface 120 has projecting threads and thread parts 130 e-spectively in the form of loops. The loops may be velour loops, floats and/or overdimensional threads. The loops are formed by threads 132 having, on their thread surfaces, anchoring structures 134 which generally project from the thread bodies. In principle, the anchoring structures 134 may be formed in any desired arrangements and configurations on the thread bodies. Fig. 1A shows by way of example an unidirectional formation of the anchoring structures on the thread surfaces wherein two sets of anchoring structures are formed in the circumferential direction of the threads 132, preferably offset by about 180° relative to each other. The projecting thread loops 130 are able to bore their way, via fine and low-irritation puncture channels, into a surrounding region of tissue, causing the implant 100 to become anchored. The anchoring structures 134 are constructed as barbs and in turn bore their way into a surrounding region of tissue and thereby provide, with particular advantage, for a slippage-resistant anchoring of the implant 100. Medium- to long-term anchoring of the implant 100 can also be achieved by the fact that body cells from a surrounding region of tissue can grow through the thread loops 130. The main surface 120 with the thread loops 132 projecting therefrom preferably faces the abdominal wall of a patient following an interperitoneal implantation.

Fig. 1B depicts a preferred embodiment of an inventive implant 100' with two mutually opposite main surfaces 110' and 120'. The main surface 110' preferably has a smooth, flat and/or tight construction, for example due to an appropriate coating or film. In this regard, the observations made in the figure description relating to Fig. 1A are referenced. The main surface 120' has projecting threads or thread parts 130', for example in the form of cut pile tufts. The threads or thread parts 130' are formed by threads displaying on their surface anchoring structures 134' in the form of barbs, which usually project from the thread surfaces. The threads or thread parts 130' can be produced for example by opening, more particularly cutting, the thread loops 130 depicted in Fig. 1A. Because the threads or thread parts 130' project from the main surface 120' an even better penetration of a biological tissue is possible. This in turn leads to an even better anchorability for the implant 100' and hence to a generally optimized security of implant. As for the rest, the observations made in the figure description relating to Fig. 1A are referenced in their entirety.

A particularly preferred embodiment of the invention is depicted in Fig. 1C. The implant 100" illustrated there likewise has two mutually opposite main surfaces 110" and 120". The main surface 110" similarly preferably has a smooth, flat and/or tight construction and may for example have a coating as more particularly described in the figure description relating to Fig. 1A. As an alternative, the main surface 110" may be designed as a film or foil. The main surface 120" displays projecting threads or thread parts 130", for example in the form of cut pile tufts. Unlike the threads or thread parts 130' depicted in Fig. 1B, the threads or thread parts 130" are formed from threads 132" with anchoring structures 134" in the form of barbs the anchoring structures 134" on the thread bodies being formed in a periodically repeating, more particularly alternating, orientation. The threads or thread parts 130" can be produced by opening, more particularly cutting, corresponding thread loops, the tops of which are shown dotted in Fig. 1C, such that the anchoring structures 134" of the threads or thread parts 130" face unidirectionally in the direction of the implant 100" or in the direction of the main surface 120". If the threads or thread parts 130" then bore their way into a biological tissue, the anchoring structures 134" will not offer any additional resistance, additionally reducing the traumatization of biological tissue as the implant 100" is being anchored or secured, which is particularly advantageous. As for the rest, the observations made in the figure description relating to Fig. 1A are again referenced in their entirety.

## Claims

1. A surgical implant (100) in the form of a textile fabric having at least two, in particular two, mutually opposite main surfaces (110,120), more particularly for management of hernias, comprising threads (130) having an elongate body and having a multiplicity of anchoring structures (134) for anchoring in biological, more particularly human and/or animal, tissues, the anchoring structures projecting from the body, **characterized in that** the anchoring structures are formed as cuts on the thread bodies.

2. A surgical implant according to claim 1, **characterized in that** the threads or parts of the threads at least partially project from the textile fabric.

3. A surgical implant according to claim 1 or 2, **characterized in that** the threads or parts thereof at least partially project from the textile fabric in the form of loops, more particularly in the form of velour loops, floats and/or overdimensioned threads.

4. A surgical implant according to any one of the preceding claims, **characterized in that** the threads or parts of the threads at least in part project from the textile fabric as cut piles, cut pile tufts, preferably as opened velour loops, pile yarns, plush or combinations thereof.

5. A surgical implantat according to any one of the preceding claims, **characterized in that** the threads or parts of the threads are tightly connected with the textile fabric, in particular with at least one main surface thereof.

6. A surgical implant according to any one of the preceding claims, **characterized in that** the threads or parts thereof at least in part project from at least one of the two main surfaces.

7. A surgical implant according to any one of the preceding claims, **characterized in that** the threads or parts thereof project from at least one of the two main surfaces of the textile fabric in a row shaped arrangement, in an offset arrangement, in a zigzag-shaped arrangement, island-like arrangement, in a spiral-shaped arrangement, in a serpentine-shaped arrangement, in a meander-shaped arrangement, in an adventitious arrangement and/or in a random arrangement.

8. A surgical implant according to any one of the preceding claims, **characterized in that** the anchoring structures are formed in the manner of barbs on the thread bodies.

9. A surgical implant according to any one of the preceding claims, **characterized in that** the textile fabric, in particular threads thereof, is formed of an absorbable and/or non-absorbable polymeric material, preferably selected from the group consisting of polylactide, polyglycolide, poly-ε-caprolactone, polytrimethylene carbonate, poly-paradioxanone, polyhydroxybutyrate, in particular poly-3-hydroxybutyrate and/or poly-4-hydroxybutyrate, polyolefins, in particular polyethylene, polypropylene, polyvinylidene difluoride, polytetrafluoropropylene, polyhexafluoropropylene and/or polytetrafluoroethylene, polyesters, polyamides, polyurethanes, copolymers thereof, terpolymers thereof and mixtures thereof.

10. A surgical implant according to any one of the preceding claims, **characterized in that** the threads are present together with other threads which have no anchoring structures on their thread bodies in a conjoined textile construction, more particularly in an Atlas, filet, tulle, mesh or openwork construction.

11. A surgical implant according to any one of the preceding claims, **characterized in that** the textile fabric, in particular at least one main surface of the textile fabric, is formed as a woven fabric, non-woven fabric, felt or interlooped fabric, braided fabric, knit fabric, in particular warp knit fabric, preferably formed-loop knit fabric, more preferably formed-loop warp-knit fabric.

12. A surgical implant according to one of the preceding claims, **characterized in that** the textile fabric, in particular a main surface thereof, comprises a coating or film, preferably having anti-adhesive properties.

13. A surgical implant according to any one of the preceding claims, **characterized in that** the threads, which have the anchoring structures, interconnect the two mutually opposite main surfaces of the textile fabric.

14. A surgical implant according to any one of the preceding claims, **characterized in that** the textile fabric is designed as a spacer textile fabric, in particular a spacer knit fabric.

15. A method of forming an implant according to any one of the preceding claims, which method comprises forming a textile fabric using, preferably at least in part, threads having an elongate body and having a multiplicity of anchoring structures projecting from the body.

## Patentansprüche

1. Chirurgisches Implantat (100) in Form eines textilen Flächengebildes mit mindestens zwei, bevorzugt zwei, sich gegenüberliegenden Hauptflächen (110, 120), insbesondere zur Versorgung von Hernien, umfassend Fäden (130) mit einem länglichen Körper und mit einer Vielzahl Verankerungsstrukturen (134) zum Verankern in biologischen, insbesondere menschlichen und/oder tierischen Geweben, wobei die Verankerungsstrukturen vom Körper abstehen, **dadurch gekennzeichnet, dass** die Verankerungsstrukturen als Einschnitte auf den Fadenkörpern ausgebildet sind.

2. Chirurgisches Implantat nach Anspruch 1, **dadurch gekennzeichnet, dass** die Fäden oder Teile der Fäden mindestens teilweise von dem textilen Flächengebilde abstehen.

3. Chirurgisches Implantat nach Anspruch 1 oder 2, **dadurch gekennzeichnet, dass** die Fäden oder Teile davon mindestens teilweise in Form von Schlingen, insbesondere in Form von Veloursschlingen, Flottungen und/oder Überfütterungen, von dem textilen Flächengebilde abstehen.

4. Chirurgisches Implantat nach einem der vorhergehenden Ansprüche, **dadurch gekennzeichnet, dass** die Fäden oder Teile der Fäden mindestens teilweise als Schnittflore, Florfäden, vorzugsweise als geöffnete Veloursschlingen, Polfäden, Plüsch oder Kombinationen davon, von dem textilen Flächengebilde abstehen.

5. Chirurgisches Implantat nach einem der vorhergehenden Ansprüche, **dadurch gekennzeichnet, dass** die Fäden oder Teile der Fäden mit dem textilen Flächengebilde, insbesondere mit mindestens einer seiner Hauptflächen, fest verbunden sind.

6. Chirurgisches Implantat nach einem der vorhergehenden Ansprüche, **dadurch gekennzeichnet, dass** die Fäden oder Teile davon mindestens teilweise von zumindest einer der beiden Hauptflächen abstehen.

7. Chirurgisches Implantat nach einem der vorhergehenden Ansprüche, **dadurch gekennzeichnet, dass** die Fäden oder Teile davon von zumindest einer der beiden Hauptflächen des textilen Flächengebildes in einer reihenförmigen Anordnung, einer versetzten Anordnung, einer zickzackförmigen Anordnung, einer inselförmigen Anordnung, einer spiralförmigen Anordnung, einer serpentinenförmigen Anordnung, einer mäanderförmigen Anordnung, einer adventiven Anordnung und/oder einer zufälligen Anordnung abstehen.

8. Chirurgisches Implantat nach einem der vorhergehenden Ansprüche, **dadurch gekennzeichnet, dass** die Verankerungsstrukturen nach Art von Widerhaken auf den Fadenkörpern ausgebildet sind.

9. Chirurgisches Implantat nach einem der vorhergehenden Ansprüche, **dadurch gekennzeichnet, dass** das textile Flächengebilde, insbesondere die Fäden darin, aus einem resorbierbaren und/oder nicht resorbierbaren Polymermaterial, bevorzugt ausgewählt aus der Gruppe bestehend aus Polylactid, Polyglykolid, Poly-ε-caprolacton, Polytrimethylencarbonat, Poly-para-dioxanon, Polyhydroxybutyrat, insbesondere Poly-3-hydroxybutyrat und/oder Poly-4-hydroxybutyrat, Polyolefinen, insbesondere Polyethylen, Polypropylen, Polyvinylidendifluorid, Polytetrafluorpropylen, Polyhexafluorpropylen und/oder Polytetrafluorethylen, Polyestern, Polyamiden, Polyurethanen, Copolymeren davon, Terpolymeren davon und Mischungen davon, gebildet sind.

10. Chirurgisches Implantat nach einem der vorhergehenden Ansprüche, **dadurch gekennzeichnet, dass** die Fäden zusammen mit anderen Fäden, die keine Verankerungsstrukturen auf ihren Fadenkörpern besitzen, in einer gemeinsamen textilen Bindung, insbesondere in einer Atlas-, Filet-, Tüll-, Mesh- oder Ajourbindung, vorliegen.

11. Chirurgisches Implantat nach einem der vorhergehenden Ansprüche, **dadurch gekennzeichnet, dass** das textile Flächengebilde, insbesondere mindestens eine Hauptfläche des textilen Flächengebildes, als Gewebe, Vlies, Filz oder Maschenware, Geflecht, Gewirke, insbesondere Kettengewirke, bevorzugt Trikot-Gewirke, besonders bevorzugt Trikot-Kettengewirke, ausgebildet ist.

12. Chirurgisches Implantat nach einem der vorhergehenden Ansprüche, **dadurch gekennzeichnet, dass** das textile Flächengebilde, insbesondere eine Hauptfläche des textilen Flächengebildes, eine Beschichtung oder einen Film, bevorzugt mit Antihafteigenschaften, umfasst.

13. Chirurgisches Implantat nach einem der vorhergehenden Ansprüche, **dadurch gekennzeichnet, dass** die Fäden, die die Verankerungsstrukturen aufweisen, die beiden sich gegenüber liegenden Hauptflächen des textilen Flächengebildes miteinander verbinden.

14. Chirurgisches Implantat nach einem der vorhergehenden Ansprüche, **dadurch gekennzeichnet, dass** das textile Flächengebilde als textiles Abstandhalterflächengebilde, insbesondere als Abstandhaltergewirke, ausgebildet ist.

15. Verfahren zur Herstellung eines Implantats nach einem der vorhergehenden Ansprüche, wobei das Verfahren Ausbilden eines texilen Flächengebildes unter Verwendung von, bevorzugt mindestens teilweise, Fäden mit einem länglichen Körper und mit einer Vielzahl von Verankerungsstrukturen, die vom Körper abstehen, umfasst.

## Revendications

1. Implant chirurgical (100) sous la forme d'un tissu textile comprenant au moins deux, en particulier deux, surfaces principales mutuellement opposées (110, 120), plus particulièrement pour la gestion de hernies, comprenant des fils (130) présentant un corps allongé et comportant une multiplicité de structures d'ancrage (134) pour réaliser un ancrage dans des tissus biologiques, plus particulièrement humains et/ou animaux, les structures d'ancrage faisant saillie à partir du corps, **caractérisé en ce que** les structures d'ancrage sont formées comme des entailles sur les corps des fils.

2. Implant chirurgical selon la revendication 1, **caractérisé en ce que** les fils ou des parties des fils font saillie au moins partiellement à partir du tissu textile.

3. Implant chirurgical selon la revendication 1 ou 2, **caractérisé en ce que** les fils ou des parties de ceux-ci font saillie au moins partiellement à partir du tissu textile sous la forme de boucles, plus particulièrement sous la forme de boucles de velours, de fils flottants et/ou de fils surdimensionnés.

4. Implant chirurgical selon l'une quelconque des revendications précédentes, **caractérisé en ce que** les fils ou des parties des fils font saillie au moins en partie à partir du tissu textile sous la forme de velours coupé, de touffes de velours coupé, de préférence de boucles de velours ouvertes, de fils de velours, de peluche ou de combinaisons de ceux-ci.

5. Implant chirurgical selon l'une quelconque des revendications précédentes, **caractérisé en ce que** les fils ou des parties des fils sont étroitement connectés au tissu textile, en particulier avec au moins une surface principale de celui-ci.

6. Implant chirurgical selon l'une quelconque des revendications précédentes, **caractérisé en ce que** les fils ou des parties de ceux-ci font saillie au moins en partie à partir d'au moins une des deux surfaces principales.

7. Implant chirurgical selon l'une quelconque des revendications précédentes, **caractérisé en ce que** les fils ou des parties de ceux-ci font saillie à partir d'au moins une des deux surfaces principales du tissu textile dans un agencement en forme de rangées, dans un agencement décalé, dans un agencement en zigzag, dans un agencement en îlots, dans un agencement hélicoïdal, dans un agencement en serpentin, dans un agencement en méandres, dans un agencement adventice et/ou dans un agencement aléatoire.

8. Implant chirurgical selon l'une quelconque des revendications précédentes, **caractérisé en ce que** les structures d'ancrage sont formées à la manière de barbes sur les corps des fils.

9. Implant chirurgical selon l'une quelconque des revendications précédentes, **caractérisé en ce que** le tissu textile, en particulier des fils de celui-ci, est constitué d'une matière polymère absorbable et/ou non absorbable, qui est de préférence sélectionnée dans le groupe comprenant le polylactide, le polyglycolide, le poly-ε-caprolactone, le polytriméthylène carbonate, le poly-paradioxanone, le polyhydroxybutyrate, en particulier le poly-3-hydroxybutyrate et/ou le poly-4-hydroxybutyrate, des polyoléfines, en particulier le polyéthylène, le polypropylène, le difluorure de polyvinylidène, le polytétrafluoropropylène, le polyhexafluoropropylène et/ou le polytétrafluoroéthylène, des polyesters, des polyamides, des polyuréthanes, des copolymères de ceux-ci, des terpolymères de ceux-ci et des mélanges de ceux-ci.

10. Implant chirurgical selon l'une quelconque des revendications précédentes, **caractérisé en ce que** les fils sont présents de concert avec d'autres fils qui ne comportent pas de structures d'ancrage sur leurs corps de fils en une structure textile conjointe, plus particulièrement une structure Atlas, en filet, en tulle, en mailles ou ajourée.

11. Implant chirurgical selon l'une quelconque des revendications précédentes, **caractérisé en ce que** le tissu textile, en particulier au moins une surface principale du tissu textile, est formé comme un tissu tissé, un tissu non tissé, un tissu de feutre ou à boucles entrelacées, un tissu tressé, un tissu tricoté, en particulier un tissu tricoté à mailles jetées, de préférence un tissu tricoté à boucles, mieux encore un tissu tricoté à mailles jetées à boucles.

12. Implant chirurgical selon l'une quelconque des revendications précédentes, **caractérisé en ce que** le tissu textile, en particulier une surface principale de celui-ci, comprend un revêtement ou un film, de préférence présentant des propriétés non adhésives.

13. Implant chirurgical selon l'une quelconque des revendications précédentes, **caractérisé en ce que** les fils, qui comportent les structures d'ancrage, interconnectent les deux surfaces principales mutuellement opposées du tissu textile.

14. Implant chirurgical selon l'une quelconque des revendications précédentes, **caractérisé en ce que** le tissu textile est conçu comme un tissu textile d'écartement, et en particulier un tissu tricoté d'écartement.

15. Procédé pour former un implant selon l'une quelconque des revendications précédentes, ledit procédé comprenant la formation d'un tissu textile en utilisant, de préférence au moins en partie, des fils présentant un corps allongé et comportant une multiplicité de structures d'ancrage faisant saillie à partir du corps.
